# FASCICULE DE BREVET EUROPEEN

(11) **EP 1 261 311 B1**
(45) Date de publication et mention de la délivrance du brevet: **22.11.2006**
(21) Numéro de dépôt: 02702451.2
(22) Date de dépôt: 05.02.2002
(51) Int. Cl.: A61K 8/06, A61K 8/60, B01F 17/00

(54) **COMPOSITIONS TOPIQUES A PHASE EXTERNE HUILEUSE ET LEUR PROCEDE DE PREPARATION**
TOPISCHE ZUSAMMENSETZUNGEN MIT EINER ÄUSSEREN ÖLPHASE UND VERFAHREN ZU DEREN HERSTELLUNG
TOPICAL COMPOSITIONS WITH OUTER PHASE AND PREPARATION METHOD

(30) Priorité: 05.02.2001 FR 0101480
(43) Date de publication de la demande: 04.12.2002
(60) Demande divisionnaire: 06120718.9
(73) Titulaire: S.E.P.P.I.C., SOCIETE D'EXPLOITATION DE PRODUITS POUR LES INDUSTRIES CHIMIQUES, 75007 Paris (FR)
(72) Inventeur: AMALRIC, Chantal, F-81700 BLAN (FR); ROSO, Alicia, F-81710 SAIX (FR); MICHEL, Nelly, F-94700 MAISONS ALFORT (FR)
(74) Mandataire: Conan, Philippe Claude
(86) Numéro de dépôt international: PCT/FR2002/000430
(87) Numéro de publication internationale: WO 2002/062305

(56) Documents cités:
- WO-A-96/04894
- US-A- 5 304 334
- DATABASE CHEMICAL ABSTRACTS [en ligne] STN; access number: 115: 214 524, XP002187692 & JP 03 178917 A (POLA CHEM. IND., INC.) 2 août 1991 (1991-08-02)
- Y. KUMANO ET AL.: "Studies of water-in-oil (w/o) emulsion stabilized with amino acids or their salts" J. SOC. COSMET. CHEM., vol. 28, no. 5, - 1977 pages 285-314, XP001058465

## Description

La présente invention a pour objet une nouvelle composition topique constituée d'une phase externe huileuse et de deux phases internes aqueuses, ainsi qu'un procédé de préparation de ladite composition.

L'invention trouve notamment application dans le domaine cosmétique, pharmaceutique, vétérinaire, ou dans le domaine des détergents.

Les émulsions traditionnelles à phase externe huileuse, présentent une texture finale désagréable pour le consommateur avec sensation de gras, difficulté d'étalement et effet collant.

Il a maintenant été découvert, et c'est le fondement de l'invention, que l'addition d'une émulsion de type "eau-dans-huile" à un gel aqueux (ou vice-versa), permet l'obtention d'une émulsion à phase externe huileuse alors que l'homme du métier se serait attendu à obtenir une émulsion de type « eau-dans-huile-dans-eau » ou une séparation de phase. Une telle émulsion à phase externe huileuse présente une texture fraîche, agréable et non collante.

Ainsi, selon un premier aspect, l'invention a pour objet une composition topique constituée d'une phase externe huileuse et de deux phases internes aqueuses dont l'une est un gel.

Avantageusement, l'huile de la phase externe huileuse représente au moins 2 % en poids, de préférence de 5 à 20 % en poids, et généralement au plus 50 % en poids de la composition topique.

Selon un second aspect, la présente invention a pour objet un procédé de préparation de la composition décrite ci-dessus, ledit procédé comprenant le mélange d'une émulsion « eau-dans-huile » (que l'on appellera par la suite « émulsion primaire à phase externe huileuse») et d'un gel aqueux.

Avantageusement, ledit mélange comprend 5 à 80 % en poids, de préférence 10 à 60 % en poids de l'émulsion primaire à phase externe huileuse, et 20 à 95 % en poids, de préférence 40 à 90 % en poids de gel aqueux.

Conformément au procédé de l'invention, il importe peu que l'on introduise l'émulsion primaire à phase externe huileuse dans le gel aqueux ou le gel aqueux dans l'émulsion primaire à phase externe huileuse. Néanmoins, il peut être avantageux d'ajouter l'émulsion primaire à phase externe huileuse dans le gel aqueux, de préférence sous agitation lente.

L'émulsion primaire à phase externe huileuse comprend généralement de 5 à 90 % en poids d'huile.

Cette huile peut être choisie parmi une ou plusieurs des huiles suivantes :
- les huiles d'origine végétale, telles que l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile, de passiflore, l'huile de noisette, l'huile de palme, le beurre de karité, l'huile de noyau d'abricot, l'huile de calophyllum, l'huile de sysymbrium, l'huile d'avocat, l'huile de calendula ;
- les huiles végétales modifiées telles que les produits connus sous les dénominations Apricot Kernel Oil PEG-6 esters, Olive Oil PEG-6 esters et LABRAFIL®;
- les huiles d'origine animale, telles que le squalène, le squalane ;
- les huiles minérales, telles que l'huile de paraffine ou huile de vaseline, et les huiles minérales, notamment issues de coupes pétrolières, telles que les isoparaffines, ayant un point d'ébullition compris entre 300 et 400°C ;
- les huiles synthétiques, notamment les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les isoparaffines, les polyalphaoléfines, les polyoléfines, comme le polyisobutène les isoalcanes de synthèse comme l'isohexadecane, l'isododécane, les huiles perfluorées et les huiles de silicone. Parmi ces dernières, on peut plus particulièrement citer les diméthylpolysiloxanes, méthylphénylpolysiloxanes, les silicones modifiés par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiés par des groupements fluorés, des silicones cycliques et des silicones modifiés par des groupements alkyles.

Cette huile peut également être choisie parmi les acides gras, les alcools gras, les cires d'origine naturelle ou synthétique, et plus généralement encore tout corps gras d'origine végétale, animale ou synthétique.

L'émulsion primaire à phase externe huileuse comprend également de 1 à 25 % en poids d'un agent émulsionnant.

Parmi les émulsionnants susceptibles d'être utilisés dans le cadre de la présente invention, on citera notamment les lipoaminoacides et leurs sels ; les lipopeptides et leurs sels ; les émulsionnants siliconés non-ioniques et anioniques ; les esters de sorbitan comme par exemple le produit dénommé MONTANE® 80 ; les esters de polyglycérol comme par exemple les produits commercialisés sous la dénomination ISOLAN® GI34 et PLUROL® DIISOSTEARIQUE ; l'huile de ricin hydrogénée éthoxylée comme par exemple le produit dénommé SIMULSOL® 989 ; le stéarate de glycérol ; les polyhydroxystéarates de polyglycol ou de polyglycérol comme par exemple les produits dénommés HYPERMER®, ARLACEL® P135 et DEHYMULS® PGPH ; les esters de sorbitan éthoxylés comme par exemple les produits commercialisés sous la dénomination MONTANOV®, ARLACEL® 581 et ARLACEL® 582 ; les acylats de protéines faiblement éthoxylés (de 1 à 3 groupements OE) ; la cire d'abeille éthoxylée comme par exemple le produit dénommé APIFIL® ; les émulsionnants cationiques comme les aminoxydes, le quaternium 82 et les tensio-actifs décrits dans la demande de brevet WO 96/00719 et principalement ceux dont la chaîne grasse comprend au moins 16 atomes de carbone ; les esters de sucrose, les esters de méthylglucoside ethoxylés ou non ; les acides gras éthoxylés ; les alcools gras éthoxylés ; les émulsionnants anioniques comme le décylphosphate ou le cétéarylsulfate ; le polyoxystéarate d'aluminium, tel que par exemple le produit commercialisé sous la dénomination MANALOX® ; le stéarate de magnésium ; le stéarate d'aluminium.

Avantageusement, on utilisera un émulsionnant du type de ceux décrits dans la demande de brevet FR-A-2 790 977, en particulier les dérivés de xylose.

On pourra également utiliser avantageusement un émulsionnant à base d'alkylpolyglycosides et de diols gras, comprenant notamment :
- 5 à 95 parties en poids d'un mélange d'alkylpolyglycosides constitué des produits de réaction d'un saccharide et d'un dimerdiol ayant 36 atomes de carbone ;
- 95 à 5 parties en poids d'un dimerdiol ayant 36 atomes de carbone.

Les émulsionnants préférés, répondant à la définition ci-dessus comprennent :
- 5 à 60 parties en poids du mélange précité d'alkylpolyglycosides ; et
- 95 à 40 parties en poids de dimerdiol ayant 36 atomes de carbone.

Le mélange d'alkylpolyglycosides constitué des produits de réaction d'un saccharide et d'un dimerdiol ayant 36 atomes de carbone est en fait constitué d'un mélange en toutes proportions d'hydroxyalkylpolyglycosides (produits résultant de l'acétalisation de l'un des deux groupes hydroxyles du dimerdiol) et de polyglycosylalkylpolyglycosides (produits résultant de l'acétalisation des deux groupes hydroxyles du dimerdiol).

Ces alkylpolyglycosides peuvent être représentés, respectivement par les formules I et II suivantes :

HO-R-O(G)ₙ (I)

(G)ₘ-OR-O-(G)ₚ (II)

dans lesquelles:
G représente un reste de saccharide ;
R représente un groupe disubstitué dérivé de l'alcool dimère provenant de l'hydrogénation de l'acide dimère ;
n, m et p représentent le degré de polymérisation moyen de chaque reste de saccharide.

Le produit connu sous la dénomination "acide dimère" est un acide dibasique ayant 36 atomes de carbone dont le composé majoritaire peut être représenté par la formule :

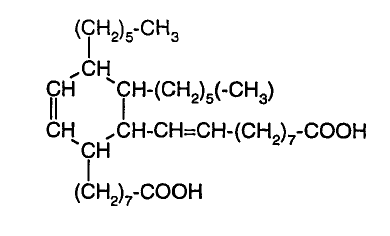

Les alkylpolyglycosides précités peuvent comporter, à titre de reste de saccharide, un reste de glucose ou dextrose, fructose, galactose, mannose, ribose, xylose, de préférence un reste de glucose ou de xylose.

Il est en outre à noter que chaque unité de la partie polyoside des alkylpolyglycosides précités peut être sous forme anomérique α ou β, et le reste de saccharide peut être de type furanoside ou pyranoside.

Le degré de polymérisation moyen de chaque reste de saccharide est généralement compris entre 1,05 et 2,5, de préférence encore entre 1,1 et 2.

L'expression "alkylpolyglycoside" utilisée dans le cadre de la présente demande désigne donc indifféremment un alkylmonooside (degré de polymérisation égal à 1) ou un alkylpolyglycoside (degré de polymérisation supérieur à 1).

Le dimerdiol utilisé pour la préparation de l'émulsionnant ci-dessus est un diol provenant de l'hydrogénation de l'acide dimère.

Il est notamment commercialisé par la Société COGNIS sous la dénomination SPEZIOL^{®} C 36/2.

Ce composé, en raison de son origine, peut contenir des proportions mineures d'impuretés. De telles impuretés peuvent être présentes en des quantités allant jusqu'à 30 % en poids du poids total de diol.

Par conséquent, les émulsionnants à base d'alkylpolyglycosides et de diols gras peuvent comprendre, en des proportions mineures correspondantes, de telles impuretés, ou les produits de réaction de ces impuretés avec un saccharide.

Les émulsionnants à base d'alkylpolyglycosides et de diols gras utilisables dans le cadre de la présente invention peuvent être préparées par simple mélange de leurs constituants en des proportions prédéterminées souhaitées.

A l'échelle industrielle, on les préparera de préférence selon l'une des deux voies classiquement utilisées pour la synthèse des alkylpolyglycosides, et par exemple par réaction, en milieu acide, entre le dimerdiol et un saccharide disposant d'un OH anomérique, tel que le glucose ou le dextrose.

Le cas échéant, cette synthèse pourra être complétée par des opérations de neutralisation, de filtration, de distillation ou d'extraction partielle du diol gras en excès ou de décoloration.

Il pourra également être avantageux d'utiliser un émulsionnant à base d'alkylpolyxyloside de formule :

R-O-(X)ₚ

dans laquelle :
p représente un nombre décimal compris entre 1 et 5,
X représente le reste du xylose, et
R représente un radical alkyl ramifié :

   CH(CₙH₂ₙ)(CₘH₂ₘ₊₁)-CH₂-
dans lequel m est un nombre entier compris entre 6 et 18, n est un nombre entier compris entre 4 et 18 et la somme n + m est supérieure ou égale à 14 ;
ou bien une composition consistant en un mélange d'au moins deux composés tels que définis ci-dessus ;
ou bien encore une composition comprenant
plus de 0% en poids et moins de 100% en poids, de préférence de 1 % à 60% en poids, d'un composé ou d'un mélange de composés définis ci-dessus et
plus de 0% en poids et moins de 100% en poids, de préférence de 40% à 99% en poids, d'un composé ou d'un mélange de composés de formule ROH dans laquelle R a la signification mentionnée précédemment.

La structure oligomérique (X)ₚ, peut se présenter sous toute forme d'isomérie, qu'il s'agisse d'isomérie optique, d'isomérie géométrique ou d'isomérie de position ; elle peut aussi représenter un mélange d'isomères.

Dans la formule R-O-(X)ₚ, le groupe R-O- est lié à X par le carbone anomérique du reste saccharide, de manière à former une fonction acétal.

p, qui représente le degré moyen de polymérisation du saccharide, est plus particulièrement compris entre 1 et 2,5 et tout particulièrement entre 1 et 2,0.

Le composé de formule R-O-(X)ₚ peut être préparé en faisant réagir un composé de formule :

HO-X

avec un excès d'alcool gras de formule ROH, puis élimination de l'alcool gras n'ayant pas réagi.

Dans le procédé tel que défini ci-dessus, la réaction de formation de l'alcool ROH est effectuée en présence de catalyseurs acides forts, tels que par exemple les acides minéraux comme l'acide sulfurique, l'acide hypophosphoreux ou un mélange de ces acides.

Selon une variante du procédé tel que défini précédemment, le xylose de formule HO-X est mis à réagir avec un alcool de formule R,-OH, dans laquelle R, comporte de 1 à 4 atomes de carbone et plus particulièrement, avec le butanol, pour conduire à l'acétal de formule R₁O-(X)ₚ, qui subit ensuite une trans-acétalisation par un excès d'alcool de formule ROH avec distillation de l'alcool de formule R₁OH formé puis élimination de l'alcool de formule ROH n'ayant pas réagi.

Dans ce procédé et sa variante, tels que décrits précédemment, l'élimination de l'alcool de formule ROH n'ayant pas réagi, est effectuée selon des méthodes connues de l'homme du métier comme, par exemple, la distillation, la distillation sur film mince, la distillation moléculaire ou l'extraction par solvants.

L'émulsion primaire à phase externe huileuse peut également comprendre un agent stabilisant.

Parmi les agents stabilisants susceptibles d'être utilisés dans le cadre de la présente invention, on peut citer l'huile de ricin hydrogénée ; les cires végétales ou animales comme par exemple la cire d'abeille et la cire de carnauba ; l'acide stéarique ; les silices hydrophobes ; les copolymères polyéthylèneglycol-alkylglycol comme le PEG-45 dodécylglycol copolymère tel que le produit commercialisé sous la dénomination ELFACOS ST 9® ; les polymères tels que les produits commercialisés sous la dénomination KRATON® ; les cires minérales comme l'ozokérite ; les argiles comme l'hectorite ou la bentonite ; les amidons modifiés hydrophobes comme par exemple le produit commercialisé sous la dénomination DRY FLOW PC ®.

L'émulsion primaire à phase externe huileuse comprend aussi avantageusement un ou plusieurs sel minéraux, comme par exemple le chlorure de magnésium, le sulfate de magnésium ou le chlorure de sodium, en une quantité allant de 0,1 % à 5 % en poids.

Les émulsions primaires à phase externe huileuse conformes à la présente invention, peuvent être préparées par simple dispersion, à une température comprise entre 15°C et 90°C, de la phase aqueuse dans la phase huileuse, en présence du ou des émulsionnants, et éventuellement du ou des stabilisants.

D'une façon connue en soi, ces émulsions peuvent en outre comprendre un ou plusieurs composés choisis parmi les humectants, comme par exemple la glycérine, les conservateurs comme par exemple les produits connus sous la dénomination SEPICIDE®, les colorants, les parfums, les actifs cosmétiques, les filtres solaires minéraux ou organiques, les charges minérales comme les oxydes de fer, oxydes de titane et le talc, les charges synthétiques comme les nylons et les poly(méthacrylate de méthyle) réticulés ou non, les élastomères silicone, les séricites et les extraits de plantes.

Ces composés pourront être introduits dans la phase aqueuse ou dans la phase huileuse, selon leur affinité pour ces phases; soit au cours de la phase de dispersion précitée, soit en ce qui concerne les composés sensibles à la température, postérieurement au cours de la phase de refroidissement dans le cas où la dispersion est réalisée à chaud.

Comme on l'a précisé précédemment, l'émulsion primaire à phase externe huileuse peut être ajoutée au gel aqueux ou inversement. Le gel aqueux est obtenu en gélifiant une phase aqueuse par un polymère. Ledit polymère est avantageusement présent en une quantité comprise entre 0,02 et 10 % en poids, de préférence entre 0,4 et 8 % en poids du gel aqueux.

Parmi les polymères susceptibles d'être utilisés dans le cadre de la présente invention, on citera notamment les homopolymères ou copolymères d'acide acrylique, de dérivés d'acide acrylique, d'acrylamide, de l'acide acrylamidométhanepropanesulfonique, de monomère vinylique, de chlorure de triméthylaminoéthylacrylate comme par exemple les produits commercialisés sous la dénomination CARBOPOL®, PEMULEN©, SIMULGEL©A, SIMULGEL ®NS, SIMULGEL®EPG, SIMULGEL®EG, LUVIGEL®EM, SALCARE®SC91, SALCARE®SC92, SALCARE®SC95, SALCARE®SC96, FLOCARE®ET100, HISPAGEL®, SEPIGEL®305, SEPIGEL®501, SEPIGEL®502, FLOCARE®ET58, STABILEZE®06 ; les hydrocolloïdes d'origine végétale ou biosynthétique comme par exemple la gomme de xanthane, la gomme de karaya, les carraghénates, les alginates ; les silicates ; la cellulose et ses dérivés; l'amidon et ses dérivés hydrophiles.

Le gel aqueux possède une viscosité supérieure à 2000 cPs, de préférence supérieure à 20 000 cPs, mesurée sur un viscosimètre BROOKFIELD LV 6 rpm.

La composition topique conforme à l'invention possède une texture fraîche, agréable et non collante.

De ce fait, la composition de l'invention peut être utilisée avantageusement dans une préparation cosmétique, pharmaceutique ou vétérinaire. La composition de l'invention peut également être utilisée dans une préparation détergente.

L'invention sera illustrée par les exemples suivants.

### EXEMPLE 1

On prépare une composition selon le mode opératoire suivant :
a) on chauffe à 80°C une phase huileuse « A » et une phase aqueuse « B » ;
b) on forme une émulsion primaire « C » à phase externe huileuse en mélangeant la phase aqueuse « B » dans la phase huileuse « A » en maintenant une agitation suffisante jusqu'à complet refroidissement ;
c) on forme un gel aqueux « D » de viscosité 80 000 cPs, mesurée au viscosimètre BROOKFIELD LV, 6 rpm, par dispersion du polymère dans la phase aqueuse ;
d) on incorpore l'émulsion dans le gel (ou le gel dans l'émulsion, cf. Tableau 1) par simple agitation manuelle, dans un rapport pondéral gel/émulsion de 70/30.

| Emulsion primaire « C » | | Gel aqueux « D » | |
|---|---|---|---|
| MONTANOV® WO18 | 8% | Carbomer | 0,4 % |
| Polyisobutène | 19 % | Eau qsp | 100 % |
| A Huile de paraffine | 21 % | SEPICIDE® HB | 0,2 % |
| ELFACOS® ST9 | 2 % | Triéthanolamine | 0,5 % |
| SEPICIDE® HB | 1 % | | |
| Eau qsp | 100 % | | |
| B Glycérine | 5 % | | |
| MgSO₄.7H₂O | 0,7 % | | |

On obtient une composition à phase externe huileuse, comme cela est confirmé par la mesure de la conductivité (< 5 µS.cm), et qui comprend en outre deux phases internes aqueuses dont l'une est un gel.

La présence de ces deux phases aqueuses est mise en évidence de la manière suivante : lors de la préparation de l'émulsion primaire, on utilise une phase aqueuse « B » qui comprend un colorant hydrophile, comme par exemple le colorant rose commercialisé par la Société WACKHERR sous la dénomination W4506. Lorsqu'on observe la composition finale au microscope, on identifie clairement les deux phases dispersées, l'une présentant une coloration rose, l'autre étant incolore.

**Tableau 1**

| Mode opératoire | Résultat et propriétés |
|---|---|
| Introduction de | - composition à phase huileuse externe |
| l'émulsion primaire | - 2 phases aqueuses internes observées au |
| « C » dans le gel aqueux | microscope |
| « D »* | - teneur en huile : 12 % en poids |
| Introduction du gel | -composition à phase huileuse externe |
| aqueux « D » dans | -2 phases aqueuses internes observées au |
| l'émulsion primaire | microscope |
| « C »* | - teneur en huile : 12 % en poids |

| | |
|---|---|
| * sous agitation de type ancre pendant 10 mn à 300 tr/mn | |

### EXEMPLE COMPARATIF

a) Composition obtenue selon l'exemple 1

| | |
|---|---|
| MONTANOV® WO18 | 2,4 % |
| Polyisobutène | 5,7 % |
| Huile de paraffine | 6,3 % |
| ELFACOS® ST9 | 0,6 % |
| SEPICIDE® HB | 0,44 % |
| Eau qsp | 100 % |
| Carbomer | 0,28 % |
| Triéthanolamine | 0,35 % |
| MgSO₄.7H₂O | 0,21 % |
| Glycérine | 1,5 % |

b) A titre de comparaison, on prépare séparément une émulsion « eau-dans-huile » de manière conventionnelle, à partir des phases huileuse et aqueuse ci-après:

| Phase huileuse | | Phase aqueuse | |
|---|---|---|---|
| MONTANOV® WO18 | 8% | MgSO₄.7H₂O | 0,7 % |
| Polyisobutène | 5,7 % | Glycérine | 1,5 % |
| Huile de paraffine | 6,3 % | Eau qsp | 100 % |
| ELFACOS® ST9 | 2 % | | |
| SEPICIDE® HB | 0,44 % | | |

| | | | |
|---|---|---|---|
| NB : On notera que, si les concentrations en émulsionnant et en stabilisant ont été augmentées (par rapport à l'émulsion du a)) afin d'obtenir une stabilité suffisante de l'émulsion, les concentrations en huiles sont identiques. | | | |

On obtient bien une émulsion à phase externe huileuse (conductivité < 5 µS.cm).

La composition selon l'invention et l'émulsion « comparative » ont été soumises à une évaluation sensorielle par un jury de 20 personnes. Les résultats sont présentés dans le Tableau 2.

**Tableau 2**

| Notation sur 10 (moyenne du jury) | Invention | Comparatif |
|---|---|---|
| Facilité d'étalement (critère positif) | 8 | 2 |
| Sensation de fraîcheur (critère positif) | 8,5 | 3 |
| Résidu (critère négatif) | 2 | 7 |
| Collant (critère négatif) | 1 | 8 |

### EXEMPLE 2

On répète la procédure de l'exemple 1, en utilisant l'émulsion primaire et le gel aqueux (de viscosité 70 000 cPs, Brookfield LV 6 rpm) ci-après dans un rapport pondéral de 50/50 :

| Emulsion primaire | | Gel aqueux | |
|---|---|---|---|
| Diméthicone copolyol | 2 % | SIMULGEL® EG | 2 % |
| Cyclométhicone DC® 345 | 23 % | Eau | 98 % |
| (Dow Corning) | | | |
| LANOL® 99 | 5 % | | |
| (isononanoate d'isononyle) | | | |
| SEPICIDE® HB | 0,3% | | |
| Eau qsp | 100 % | | |
| Glycérine | 5 % | | |
| Chlorure de sodium | 2 % | | |
| SEPICIDE® Cl | 0,2 % | | |

On obtient une composition à phase externe huileuse (conductivité < 5 µS.cm), qui comprend deux phases internes aqueuses dont l'une est un gel. La teneur en huile est de 15 % en poids.

### EXEMPLE 3

On prépare tout d'abord un émulsionnant de type alkylpolyglycoside de la manière suivante :
792,8 g d'alcool dimère en C₃₆ (commercialisé par la Société COGNIS sous la dénomination SPEZIOL C_{36/2}) sont introduits dans un réacteur en verre de deux litres, équipé d'une agitation mécanique efficace, d'un système de chauffage par double enveloppe, d'un condensateur et d'une sonde de température.

L'alcool dimère est chauffé à 90°C et 112,0 g de xylose sont dispersés dans le milieu réactionnel puis homogénéisés à 90°/95°C pendant 15 minutes.

1,90 g d'acide sulfurique à 98 % et 1,31 g d'acide hypophosphoreux à 50 % sont ajoutés et le mélange réactionnel est maintenu à 95°C pendant 4 heures sous vide partiel, avec un barbotage d'azote.

Après refroidissement à 80°C, le mélange est neutralisé de façon à atteindre une valeur du pH d'une solution à 5 % du milieu réactionnel d'environ 7,1 par ajout d'une solution de borohydrure de sodium dans la lessive de soude.

Le produit résultant se présente sous la forme d'un liquide limpide et visqueux, présentant une teneur en alcool libre de 50 % en poids. Ce produit est dénommé « APG1 » dans ce qui suit.

On répète ensuite la procédure de l'exemple 1, en utilisant l'émulsion primaire et le gel aqueux ci-après dans un rapport pondéral de 50/50 :

| Emulsion primaire | | Gel aqueux | |
|---|---|---|---|
| APG1 | 8% | SIMULGEL® 600 | 2 % |
| ELFACOS® ST9 | 2 % | Eau | 98 % |
| Huile de paraffine | 30 % | | |
| LANOL® 1688 | 10% | | |
| (éthylhexanoate de | | | |
| cétéaryle) | | | |
| Eau qsp | 100 % | | |
| Glycérine | 5 % | | |
| MgSO₄.7H₂O | 0,7 % | | |

On obtient une composition à phase externe huileuse (conductivité < 5 µS.cm), qui comprend deux phases internes aqueuses dont l'une est un gel. La teneur en huile est de 20 % en poids.

### EXEMPLE 4

On répète la procédure de l'exemple 1, en utilisant l'émulsion primaire et le gel aqueux ci-après, dans un rapport pondéral gel/émulsion de 80/20 :

| Emulsion primaire | | Gel aqueux | |
|---|---|---|---|
| APX isostéarylique* | 10 % | SIMULGEL® EG | 1,5 % |
| Squalane | 40 % | Eau | 98,5 % |
| Eau qsp | 100 % | | |
| Glycérine | 5 % | | |
| MgSO₄.7H₂O | 0,7 % | | |

| | | | |
|---|---|---|---|
| * préparé selon le mode opératoire décrit à l'exemple 1 de la demande FR-A-2 790 977, en remplaçant le glucose par du xylose | | | |

On obtient une composition à phase externe huileuse (conductivité < 5 µS.cm), qui comprend deux phases internes aqueuses dont l'une est un gel. La teneur en huile est de 8 %.

## Revendications

1. Composition topique, **caractérisée en ce qu'**elle est constituée d'une phase externe huileuse et de deux phases internes aqueuses dont l'une est un gel, le gel aqueux étant obtenu en gélifiant une phase aqueuse par un polymère.

2. Composition selon la revendication 1, **caractérisée en ce** ledit polymère est un homopolymère ou copolymère d'acide acrylique, de dérivé(s) d'acide acrylique, d'acrylamide, ou de l'acide acrylamidométhanepropanesulfonique.

3. Composition selon la revendication 1 ou 2, **caractérisée en ce que** l'huile de la phase externe huileuse représente au moins 2 % en poids, de préférence de 5 à 20 % en poids, et au plus 50 % en poids, de la composition topique.

4. Composition selon l'une quelconque des revendications 1, 2 ou 3, **caractérisée en ce que** la composition comprend au moins un émulsionnant choisi dans le groupe constitué par : les lipoaminoacides et leurs sels; les lipopeptides et leurs sels ; les esters de sorbitan; les esters de polyglycérol; l'huile de ricin hydrogénée éthoxylée ; le stéarate de glycérol; les polyhydroxystéarates de polyglycol ou de polyglycérol; les esters de sorbitan éthoxylés ; les acylats de protéines faiblements éthoxylés ; la cire d'abeille éthoxylée ; les émulsionnants cationiques ; les esters de méthylglucoside; les acides gras éthoxylés ; les alcools gras éthoxylés ; le décylphosphate ; le cétéarylsulfate ; le polyoxystéarate d'aluminium; le stéarate de magnésium ; le stéarate d'aluminium ; un émulsionnant comprenant :
- 5 à 95 parties en poids d'un mélange d'alkylpolyglycosides constitué des produits de réaction d'un saccharide et d'un dimerdiol ayant 36 atomes de carbone, et
- 95 à 5 parties en poids d'un dimerdiol ayant 36 atomes de carbone;
un émulsionnant à base d'alkylpolyxyloside de formule :
R-O-(X)ₚ
dans laquelle :
p représente un nombre décimal compris entre 1 et 5,
X représente le reste du xylose, et
R représente un radical alkyl ramifié :
CH(CₙH₂ₙ₊₁(CₘH₂ₘ₊₁)-CH₂-
dans lequel m est un nombre entier compris entre 6 et 18, n est un nombre entier compris entre 4 et 18 et la somme n + m est supérieure ou égale à 14 ;
une composition consistant en un mélange d'au moins deux émulsionnants de formule R-O-(X)ₚ telles que définie plus haut;
une composition comprenant :
plus de 0% en poids et moins de 100% en poids, de préférence de 1% à 60% en poids, d'un composé ou d'un mélange de composés de formule R-O-(X)ₚ définis ci-dessus, et
plus de 0% en poids et moins de 100% en poids, de préférence de 40% à 99% en poids, d'un composé ou d'un mélange de composés de formule ROH dans laquelle R a la signification mentionnée précédemment.

5. Composition selon l'une quelconque des revendications 1, 2, 3 ou 4, dans laquelle phase externe huileuse est constituée d'au moins une huile choisie dans le groupe constitué par : les huiles d'origine végétale ; les huiles végétales modifiées ; les huiles d'origine animale ; les huiles synthétiques choisies dans le groupe constitué par : les esters d'acides gras, les esters dérivés d'acide lanolique, les monoglycérides, diglycérides et triglycérides d'acides gras, les alkylbenzoates, les isoparaffines, les polyalphaoléfines, les polyoléfines et les huiles perfluorées; les acides gras; les alcools gras; les cires d'origine naturelle ou synthétique.

6. Procédé de fabrication d'une composition selon l'une quelconque des revendications 1, 2, 3, 4 ou 5, **caractérisé en ce qu'**il comprend le mélange d'une émulsion primaire à phase externe huileuse et d'un gel aqueux, le gel aqueux étant obtenu en gélifiant une phase aqueuse par un polymère.

7. Procédé selon la revendication 6, **caractérisé en ce qu'**on mélange 5 à 80 % en poids, de préférence 10 à 60 % en poids de l'émulsion primaire à phase externe huileuse avec 20 à 95 % en poids, de préférence 40 à 90 % en poids de gel aqueux.

8. Procédé selon la revendication 6 ou 7, **caractérisé en ce qu'**on introduit l'émulsion primaire à phase externe huileuse dans le gel aqueux.

9. Procédé selon la revendication 6 ou 7, **caractérisé en ce qu'**on introduit le gel aqueux dans l'émulsion primaire à phase externe huileuse.

10. Procédé selon la revendication l'une quelconque des revendications 6 à 9, **caractérisé en ce que** ledit polymère est présent en une quantité comprise entre 0,02 et 10 % en poids, de préférence entre 0,4 et 8 % en poids du gel aqueux.

11. Procédé selon l'une des revendications 6 à 10, **caractérisé en ce que** le gel aqueux a une viscosité supérieure à 2000 cPs, de préférence supérieure à 20 000 cPs, mesurée au Brookfield LV 6 rpm.

12. Procédé selon l'une des revendications 6 à 11, **caractérisé en ce que** l'émulsion primaire à phase externe huileuse comprend de 5 à 90 % en poids d'huile, notamment 5 à 90 % en poids d'une ou plusieurs huiles choisies parmi les huiles végétales, les huiles animales, les huiles minérales et les huiles synthétiques.

13. Procédé selon l'une des revendications 6 à 12, **caractérisé en ce que** l'émulsion primaire à phase externe huileuse comprend de 1 à 25 % en poids d'un agent émulsionnant.

14. Préparation cosmétique, pharmaceutique, vétérinaire ou détergente, **caractérisée en ce qu'**elle comprend une composition selon l'une des revendications 1 à 5, ou une composition obtenue par le procédé selon l'une des revendications 6 à 13.

15. Préparation cosmétique selon la revendication 14, **caractérisée en ce qu'**elle comprend un ou plusieurs filtres solaires minéraux ou organiques.

## Claims

1. Topical composition, **characterized in that** it is composed of an outer oily phase and of two inner aqueous phases, one of which is a gel, the aqueous gel being obtained by gelling an aqueous phase with a polymer.

2. Composition according to Claim 1, **characterized in that** said polymer is a homopolymer or copolymer of acrylic acid, acrylic acid derivative(s), acrylamide or acrylamidomethanepropanesulfonic acid.

3. Composition according to Claim 1 or 2, **characterized in that** the oil of the outer oily phase represents at least 2% by weight, preferably 5 to 20% by weight, and not more than 50% by weight, of the topical composition.

4. Composition according to any of Claims 1, 2 or 3, **characterized in that** the composition comprises at least one emulsifier selected from the group consisting of lipoamino acids and their salts, lipopeptides and their salts, sorbitan esters, polyglycerol esters, ethoxylated hydrogenated castor oil, glycerol stearate, polyglycol or polyglycerol polyhydroxystearates, ethoxylated sorbitan esters, protein acylates with a low degree of ethoxylation, ethoxylated beeswax, cationic emulsifiers, methylglucoside esters, ethoxylated fatty acids, ethoxylated fatty alcohols, decyl phosphate, cetearyl sulphate, aluminium polyoxystearate, magnesium stearate, aluminium stearate, an emulsifier comprising:
- 5 to 95 parts by weight of an alkylpolyglycoside mixture consisting of the reaction products of a saccharide and a dimerdiol having 36 carbon atoms, and
- 95 to 5 parts by weight of a dimer diol having 36 carbon atoms,
an emulsifier based on alkylpolyxyloside of the formula:
R-O-(X)ₚ
in which
p represents a decimal number between 1 and 5,
X represents the xylose residue and
R represents a branched alkyl radical:
CH (CₙH₂ₙ₊₁) (CₘH₂ₘ₊₁)- CH₂-
in which m is an integer between 6 and 18, n is an integer between 4 and 18 and the total of n + m is 14 or above,
a composition consisting of a mixture of at least two emulsifiers of the formula R-O-(X)ₚ as defined above, a composition comprising:
more than 0% by weight and less than 100% by weight, preferably 1 to 60% by weight, of a compound or a mixture of compounds of the formula R-O-(X)ₚ defined hereinabove, and
more than 0% by weight and less than 100% by weight, preferably 40 to 99% by weight, of a compound or a mixture of compounds of the formula ROH in which R has the meaning given above.

5. Composition according to any of Claims 1, 2, 3 or 4, in which the outer oily phase is composed of at least one oil selected from the group consisting of oils of vegetable origin, modified vegetable oils, oils of animal origin; synthetic oils selected from the group consisting of fatty acid esters, esters derived from lanolic acid, monoglycerides, diglycerides and triglycerides of fatty acids, alkylbenzoates, isoparaffins, poly-alpha-olefins, polyolefins and perfluorinated oils; fatty acids; fatty alcohols; and waxes of natural or synthetic origin.

6. Process for the manufacture of a composition according to any of Claims 1, 2, 3, 4 or 5, **characterized in that** it comprises mixing a primary emulsion with outer oily phase and an aqueous gel, the aqueous gel having been obtained by gelling an aqueous phase with a polymer.

7. Process according to Claim 6, **characterized in that** 5 to 80% by weight, preferably 10 to 60% by weight, of the primary emulsion with external oily phase are mixed with 20 to 95% by weight, preferably 40 to 90% by weight, of aqueous gel.

8. Process according to Claim 6 or 7, **characterized in that** the primary emulsion with outer oily phase is introduced into the aqueous gel.

9. Process according to Claim 6 or 7, **characterized in that** the aqueous gel is introduced into the primary emulsion with outer oily phase.

10. Process according to any one of Claims 6 to 9, **characterized in that** said polymer is present in an amount of between 0.02 and 10% by weight, preferably between 0.4 and 8% by weight, of the aqueous gel.

11. Process according to any of Claims 6 to 10, **characterized in that** the aqueous gel has a viscosity above 2000 cPs, preferably above 20 000 cPs, measured with a Brookfield LV at 6 rpm.

12. Process according to any of Claims 6 to 11, **characterized in that** the primary emulsion with outer oily phase comprises 5 to 90% by weight of oil, in particular 5 to 90% by weight of one or more oils selected among vegetable oils, animal oils, mineral oils and synthetic oils.

13. Process according to any of Claims 6 to 12, **characterized in that** the primary emulsion with outer oily phase comprises 1 to 25% by weight of an emulsifier.

14. Cosmetic, pharmaceutical, veterinary or detergent preparation, **characterized in that** it comprises a composition according to any of Claims 1 to 5 or a composition obtained by the process according to any of Claims 6 to 13.

15. Cosmetic preparation according to Claim 14, **characterized in that** it comprises one or more mineral or organic sunscreens.

## Patentansprüche

1. Topische Zusammensetzung, **dadurch gekennzeichnet, daß** sie aus einer äußeren Ölphase und zwei inneren wäßrigen Phasen, von denen eine ein Gel ist, besteht, wobei das wäßrige Gel **dadurch** erhalten wurde, daß man eine wäßrige Phase mit einem Polymer geliert.

2. Zusammensetzung nach Anspruch 1, **dadurch gekennzeichnet, daß** es sich bei dem Polymer um ein Homopolymer oder Copolymer von Acrylsäure, Acrylsäurederivat(en), Acrylamid oder Acrylamidomethanpropansulfonsäure handelt.

3. Zusammensetzung nach Anspruch 1 oder 2, **dadurch gekennzeichnet, daß** das Öl der äußeren Ölphase mindestens 2 Gew.-%, vorzugsweise 5 bis 20 Gew.-%, maximal 50 Gew.-%, der topischen Zusammensetzung ausmacht.

4. Zusammensetzung nach einem der Ansprüche 1, 2 oder 3, **dadurch gekennzeichnet, daß** die Zusammensetzung mindestens einen Emulgator aus der Gruppe Lipoaminosäuren und ihre Salze, Lipopeptide und ihre Salze, Sorbitanester, Polyglycerinester, ethoxyliertes hydriertes Rizinusöl, Glycerinstearat, Polyglycol- oder Polyglycerinpolyhydroxystearate, ethoxylierte Sorbitanester, schwach ethoxylierte Proteinacylate, ethoxyliertes Bienenwachs, kationische Emulgatoren, Methylglucosidester, ethoxylierte Fettsäuren, ethoxylierte Fettalkohole, Decylphosphat, Cetearylsulfat, Aluminiumpolyoxystearat, Magnesiumstearat, Aluminiumstearat, ein Emulgator, der folgendes enthält:
- 5 bis 95 Gewichtsteile einer Alkylpolyglycosidmischung, die aus Reaktionsprodukten eines Saccharids und eines Dimerdiols mit 36 Kohlenstoffatomen besteht, und
- 95 bis 5 Gewichtsteile eines Dimerdiols mit 36 Kohlenstoffatomen;
ein Emulgator auf Alkylpolyxylosidbasis der Formel:
R-O-(X)ₚ
in der:
p eine Dezimalzahl zwischen 1 und 5 bedeutet,
X den Xyloserest bedeutet und
R einen verzweigten Alkylrest bedeutet:
CH(CₙH₂ₙ₊₁) (CₘH₂ₘ₊₁)-CH₂-
in dem m eine ganze Zahl zwischen 6 und 18 bedeutet, n eine ganze Zahl zwischen 4 und 18 bedeutet und die Summe von n+m 14 oder darüber beträgt,
eine Zusammensetzung, die aus einer Mischung von mindestens zwei Emulgatoren der Formel R-O-(X)ₚ wie oben definiert besteht,
eine Zusammensetzung, die folgendes enthält:
mehr als 0 Gew.-% und weniger als 100 Gew.-%, vorzugsweise 1 Gew.-% bis 60 Gew.-%, einer Verbindung oder einer Mischung von Verbindungen der Formel R-O-(X)ₚ wie oben definiert und
mehr als 0 Gew.-% und weniger als 100 Gew.-%, vorzugsweise 40 Gew.-% bis 99 Gew.-%, einer Verbindung oder einer Mischung von Verbindungen der Formel ROH, in der R die oben angegebene Bedeutung aufweist,
umfaßt.

5. Zusammensetzung nach einem der Ansprüche 1, 2, 3 oder 4, in der die äußere Ölphase aus mindestens einem Öl aus der Gruppe Öle pflanzlichen Ursprungs, modifizierte pflanzliche Öle, Öle tierischen Ursprungs, synthetische Öle aus der Gruppe Fettsäureester, Ester der Lanolinsäure, Monoglyceride, Diglyceride und Triglyceride von Fettsäuren, Alkylbenzoate, Isoparaffine, Poly-alpha-olefine, Polyolefine und perfluorierte Öle, Fettsäuren, Fettalkohole, sowie Wachse natürlichen oder synthetischen Ursprungs besteht.

6. Verfahren zur Herstellung einer Zusammensetzung nach einem der Ansprüche 1, 2, 3, 4 oder 5, **dadurch gekennzeichnet, daß** man dabei eine Primäremulsion mit äußerer Ölphase und ein wäßriges Gel mischt, wobei das wäßrige Gel **dadurch** erhalten wurde, daß man eine wäßrige Phase mit einem Polymer geliert.

7. Verfahren nach Anspruch 6, **dadurch gekennzeichnet, daß** man 5 bis 80 Gew.-%, vorzugsweise 10 bis 60 Gew.-% der Primäremulsion mit äußerer Ölphase mit 20 bis 95 Gew.-%, vorzugsweise 40 bis 90 Gew.-%, wäßrigem Gel mischt.

8. Verfahren nach Anspruch 6 oder 7, **dadurch gekennzeichnet, daß** man die Primäremulsion mit äußerer Ölphase in das wäßrige Gel einbringt.

9. Verfahren nach Anspruch 6 oder 7, **dadurch gekennzeichnet, daß** man das wäßrige Gel in die Primäremulsion mit äußerer Ölphase einbringt.

10. Verfahren nach einem der Ansprüche 6 bis 9, **dadurch gekennzeichnet, daß** das Polymer in einer Menge zwischen 0,02 und 10 Gew.-%, vorzugsweise zwischen 0,4 und 8 Gew.-%, des wäßrigen Gels vorliegt.

11. Verfahren nach einem der Ansprüche 6 bis 10, **dadurch gekennzeichnet, daß** das wäßrige Gel eine mit dem Brookfield-LV-Gerät bei 6 U/min gemessene Viskosität über 2000 cPs, vorzugsweise über 20 000 cPs, aufweist.

12. Verfahren nach einem der Ansprüche 6 bis 11, **dadurch gekennzeichnet, daß** die Primäremulsion mit äußerer Ölphase 5 bis 90 Gew.-% Öl, insbesondere 5 bis 90 Gew.-% an einem oder mehreren Ölen aus der Reihe pflanzliche Öle, tierische Öle, Mineralöle und synthetische Öle, umfaßt.

13. Verfahren nach einem der Ansprüche 6 bis 12, **dadurch gekennzeichnet, daß** die Primäremulsion mit äußerer Ölphase 1 bis 25 Gew.-% eines Emulgators umfaßt.

14. Kosmetisches, pharmazeutisches oder tierärztliches Präparat oder Reinigungspräparat, **dadurch gekennzeichnet, daß** es eine Zusammensetzung nach einem der Ansprüche 1 bis 5 oder eine Zusammensetzung, die nach dem Verfahren nach einem der Ansprüche 6 bis 13 erhalten wird, umfaßt.

15. Kosmetikpräparat nach Anspruch 14, **dadurch gekennzeichnet, daß** es einen oder mehrere mineralische oder organische UV-Filter umfaßt.
